Europäisches Patentamt

**European Patent Office**

**Office européen des brevets**

(11) Veröffentlichungsnummer: **0 444 462 A1**

## EUROPÄISCHE PATENTANMELDUNG

(21) Anmeldenummer: **91101866.1**

(22) Anmeldetag: **11.02.91**

(51) Int. Cl.5: **A61M 5/44**, A61M 1/36, G01F 1/68

(30) Priorität: **14.02.90 DE 4004513**

(43) Veröffentlichungstag der Anmeldung: **04.09.91 Patentblatt 91/36**

(84) Benannte Vertragsstaaten: **AT BE CH DE ES FR GB IT LI NL**

(71) Anmelder: **MEDICAL SUPPORT GMBH Raiffeisenstrasse 2 W-6054 Rodgau 2(DE)**

(72) Erfinder: **Der Erfinder hat auf seine Nennung verzichtet**

(74) Vertreter: **Flosdorff, Jürgen, Dr. Alleestrasse 33 W-8100 Garmisch-Partenkirchen(DE)**

(54) **Verfahren und Einrichtung zum Aufheizen und zur Mengenmessung eines strömenden Fluids, insbesondere einer Flüssigkeit.**

(57) Eine strömende Flüssigkeit wie z.B. eine Infusionslösung wird mittels einer in ein Schlauchsystem integrierten elektrischen Heizkanüle (8) eine vorgegebene Temperatur aufgeheizt. Temperatursensoren (12,13) erfassen die Temperaturerhöhung in der Heizkanüle (8), während gleichzeitig der Heizenergieverbrauch der Heizkanüle ermittelt wird. Aus den Meßwerten der Temperaturerhöhung und des Heizenergieverbrauchs wird die Durchflußmenge der Flüssigkeit errechnet, wodurch eine zusätzliche, von der Heizeinrichtung (9,17) getrennte Einrichtung zur Ermittlung der Durchflußmenge entfällt.

**VERFAHREN UND ANORDNUNG ZUM MENGENMESSENDEN AUFHEIZEN EINES STRÖMENDEN FLUIDS, INSBESONDERE EINER FLÜSSIGKEIT**

Die Erfindung betrifft ein Verfahren sowie eine Anordnung zum mengenmessenden Aufheizen eines strömenden Fluids, insbesondere einer Flüssigkeit, wobei die Flüssigkeit eine elektrische Heizeinrichtung durchströmt.

Insbesondere in der Medizin stellt sich z.B. bei Infusionen, Hämofiltrationen, Hämodiafiltrationen, Plasmaaustausch und bei der Peritonealdialyse das Problem, eine strömende, sterile Flüssigkeit wie z.B. eine Infusionslösung in einen Schlauchsystem auf eine definierte Temperatur von beispielsweise 37° C aufzuheizen. Zu diesem Zweck sind verschiedene Verfahren und Vorrichtungen bekannt. Beispielweise kann eine bestimmte Länge des Schlauchsystems auf einen thermostatisierten Heizkörper gewickelt werden, oder es kann in das Schlauchsystem ein spezieller Plastebeutel mit einer Waffeleisen-ähnlichen Heizung integriert sein. Es ist ferner bekannt, ein metallisches Heizrohr in das Schlauchsystem zu integrieren, das direkt elektrisch beheizt wird.

Bei den genannten Anwendungsgebieten besteht im allgemeinen außerdem das Problem, daß die Durchflußmenge pro Zeiteinheit und die durchgeflossene Gesamtmenge der strömenden Flüssigkeit bestimmt werden müssen. Auch hierzu sind verschiedene Verfahren und Vorrichtungen bekannt, die auf der Tropfenzählung, einem Wiegen der Flüssigkeit sowie einer elektromagnetischen Strömungsmessung basieren. Allen Verfahren und Vorrichtungen zur Erfassung der Strömungsmenge ist gemeinsam, daß diese verfahrenstechnisch völlig unabhängig von dem Aufheizen der Flüssigkeit vonstatten gehen und daß eine gesonderte Vorrichtung hierzu erforderlich ist, die verhältnismäßig aufwendig ist.

Der vorliegenden Erfindung liegt die Aufgabe zugrunde, ein Verfahren und eine Anordnung der betrachteten Art anzugeben, mit dem bzw. der zuverlässig und präzise ein strömendes Fluid, vorzugsweise eine strömende Flüssigkeit auf eine vorgegebene Temperatur aufheizbar und die Durchflußmenge pro Zeiteinheit sowie die Gesamtdurchflußmenge auf einfache Weise und exakt bestimmbar ist.

Diese Aufgabe wird durch die im Kennzeichen der Patentansprüche 1 und 5 angegebenen Merkmale gelöst. Vorteilhafte Weiterbildungen der Erfindung sind in den abhängigen Ansprüchen gekennzeichnet.

Gemäß der vorliegenden Erfindung wird das strömende Fluid von einer in den Strömungskanal, vorzugsweise ein Schlauchsystem, integrierten elektrischen Heizeinrichtung aufgeheizt, wobei diese direkte elektrische Beheizung den Vorteil einer engen energetischen Kopplung und damit einer geringen Regelträgheit hat. Als elektrische Heizeinrichtung wird in einer bevorzugten Ausführungsform eine Heizkanüle verwendet.

Die vorstehend erwähnte direkte energetische Kopplung ermöglicht es erfindungsgemäß, die Durchflußmenge des Fluids aus den Heizparametern zu bestimmen. Die Grundlage hierfür bildet die Gleichung

(1)   $E = m \times c \times (T_0 - T_i)$

wobei E = Heizenergie, m = Flüssigkeitsmasse, $T_0$ = Temperatur am Ausgang der Heizeinrichtung und $T_i$ = Temperatur am Eingang der Heizeinrichtung ist. Der Proportionalitätsfaktor c ist eine fluidspezifische Konstante.

Die bis zu einem bestimmten Zeitpunkt durchgeflossene aufgeheizte Gesamtdurchflußmenge m wird dadurch bestimmt, daß die aufgewendete Energiemenge gemessen und durch die ebenfalls zu messende Temperaturdifferenz sowie durch die bekannte spezifische Konstante c dividiert wird:

$$(2) \quad m = \frac{E}{c \times (T_0 - T_i)}$$

Die Durchflußmenge pro Zeiteinheit wird dadurch bestimmt, daß Gleichung (2) durch t geteilt wird, so daß

$$(3) \quad m/t = \frac{P}{c \times (T_0 - T_i)}$$

wobei P = E/t ist.

Das erfindungsgemäße Prinzip ist nicht nur zum mengenmessenden Aufheizen einer Flüssigkeit verwendbar, sondern auch auf gasförmige Fluide anwendbar, wobei deren gasspezifische Konstante c bekannt sein oder ermittelt werden muß.

Das erfindungsgemäße Verfahren sieht vor, daß die Temperatur der Flüssigkeit vor dem Einströmen in die Heizeinrichtung und im Ausströmbereich der Heizeinrichtung gemessen und die Temperaturdifferenz bestimmt wird. Außerdem werden die für den Heizenergieverbrauch der elektrischen Heizeinrichtung maßgeblichen Daten gemessen, woraufhin der Heizenergieverbrauch aus den gemessenen und bekannten Daten der elektrischen Spannung, Stromstärke und des elektrischen Widerstandes bestimmt wird. Aus den erfaßten sowie bekannten Daten (zu den letzteren gehört die spezifische Konstante c) wird mittels einer analogen oder digitalen Auswertemöglichkeit nach den vorstehend angegebenen Gleichungen die aktuelle Durchflußmenge sowie die Gesamtdurchflußmenge errechnet.

Die Heizkanüle kann von einem Computer mittels einer Pulspaketsteuerung in Abhängigkeit von der jeweils gemessenen Ausströmtemperatur der Flüssigkeit so geregelt werden, daß ein vorgegebener Temperatur-Sollwert genauestens eingehalten wird.

Die Netzfrequenz und die Netzspannung können periodische gemessen werden. Die Heizleistung kann aus Pulsverhältnis, Spannung und Widerstand berechnet werden, während die Energiemenge aus Pulszahl, Spannung und Widerstand berechnet werden kann.

Mit großem Vorteil ist vorgesehen, daß die errechnete Durchflußmenge pro Zeiteinheit sowie die gesamte Durchflußmenge von einem Computer mit vorgegebenen Grenzwerten verglichen werden und daß bei Überschreiten der Grenzwerte ein Alarmzeichen erzeugt wird.

Bei dem erfindungsgemäßen Verfahren wird auf einfach Weise der Heizenergieverbrauch dazu herangezogen, die Durchflußmenge der aufgeheizten Flüssigkeit zu bestimmen, wobei dies wegen der direkten energetischen Kopplung bei dem verwendeten Heizverfahren mit äußerster Genauigkeit erfolgt. Somit kann zu jedem Zeitpunkt auf äußerst einfache Weise die Durchflußmenge überwacht und bestimmt werden.

Die erfindungsgemäße Anordnung enthält eine Einrichtung zur Erfassung der Temperaturerhöhung in der Heizeinrichtung, wobei je ein Temperatursensor im Einströmbereich und im Außenbereich der Heizeinrichtung angeordnet sein kann. Die Temperaturdifferenz kann auch direkt durch einen TEmperaturdifferenzsensor ermittelt werden. Die erfindungsgemäße Anordnung enthält ferner eine Einrichtung zur Erfassung des Heizenergieverbrauchs der Heizeinrichtung, d.h. zur Erfassung der maßgebenden elektrischen DAten, und und eine analoge oder digitale Einrichtung zur rechnerischen Bestimmung der Durchflußmenge der Flüssigkeit aus der Temperaturerhöhung und dem zugehörigen Heizenergieverbrauch.

Die Heizeinrichtung, die in einer Ausführungsform durch eine Heizkanüle gebildet ist, kann über elektrische Kontaktklemmen in einen Niedervolt-Regelstromkreis integriert sein, wobei der Heizstrom von einem Computer mittels einer Pulspaketsteuerung gesteuert werden kann. Dem Computer werden die zur Heizleistungsberechnung erforderlichen Daten ebenso zugeführt wie die Meßwerte der Temperatursensoren.

Erfindungsgemäß kann ferner ein Anzeigegerät angeordnet sein, das die Ausströmtemperatur der Flüssigkeit, die Durchflußmenge in g/min und die durchgeflossene Gesamtmenge in Gramm anzeigen kann. Das Anzeigegerät kann ferner mit einer optischen oder akustischen Warnanzeige ausgerüstet sein, die ein Alarmzeichen abgibt, wenn vorgegebene Grenzwerte überschritten werden.

Nachfolgend wird ein Ausführungsbeispiel der Erfindung in näheren Einzelheiten beschrieben. Dabei ist ein Gerät zur Peritonealdialyse mit der erfindungsgemäßen Anordnung ausgerüstet.

Hierzu ist eine ca. 20 cm lange Kanüle beidseitig in das Patienten-Schlauchsystem eingefügt und über elektrische Kontaktklemmen in einen Niedervolt-Regelstromkreis(max. ca. 4 V, 50 A) integriert. Dieser gestattet die Computer-gesteuerte Regelung des Heizstroms in der Kanüle mittels einer Pulspaketsteuerung. Über zwei Temperatursensoren erhält der Computer Informationen über die Ein- und Ausströmtemperaturen der Heizkanüle und regelt nach diesen Meßwerten die Heizleistung so, daß eine vorgegebene Ausgangstemperatur (37° C) konstant gehalten wird.

Zur Heizleistungsgerechnung werden Netzfrequenz und Netzspannung ebenfalls periodisch gemessen. Die zur Heizleistungsberechnung erforderlichen Daten werden dem Computer zugeführt, der mit dem Regelstromkreis und den Temperatursensoren verbunden ist.

Aus allen diesen Daten berechnet der Computer neben seiner Temperatur-Regelfunktion die folgenden aktuellen und gemittelten Parameter zur Anzeige:

Netzfrequenz und Netzspannung,

Einström-, Ausström- und Differenztemperatur,

Heizleistung aus Pulsverhältnis, Spannung und Widerstand,

Energiemenge aus Pulszahl, Spannung und Widerstand,

Durchflußmenge in g/min und

EP 0 444 462 A1

durchgeflossene Gesamtmenge in g.

Alle diese Parameter werden vom Computer laufend überwacht und mit Alarm-Grenzwerten verglichen. Dadurch ergibt sich ein hohes Sicherheitsniveau des Gerätes.

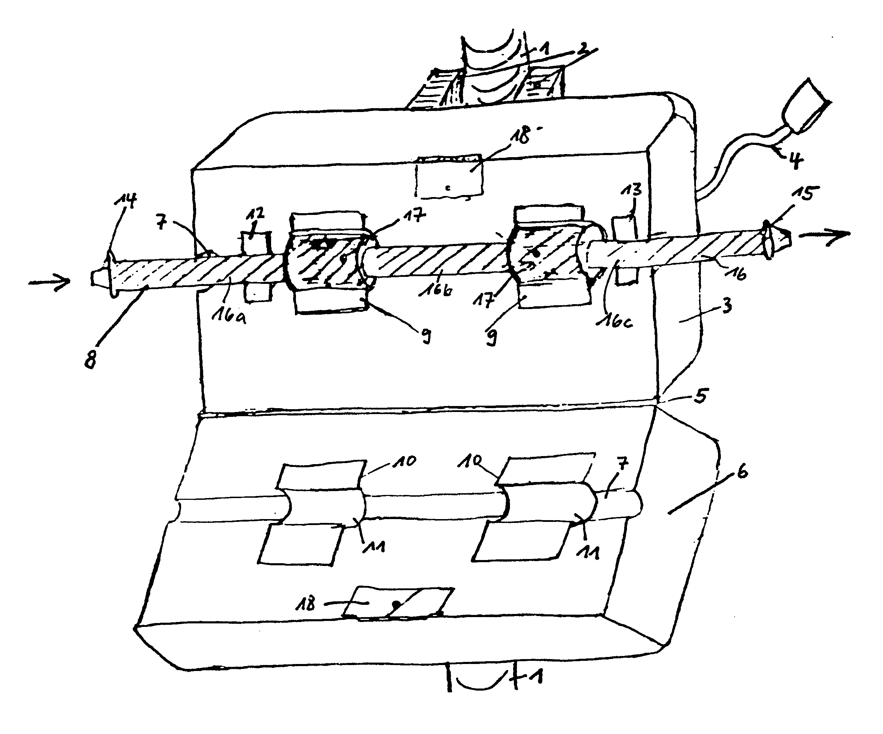

Weitere Einzelheiten der Erfindung ergeben sich aus der nachfolgenden Beschreibung einer bevorzugten Ausführungsform sowie anhand der Zeichnung, die eine Anordnung zum Aufheizen eines strömenden Fluids zeigt.

An einem Stativ 1 ist mittels Stativklemmen 2 ein Aufnahmelager 3 mit einer Stromzuführung 4 (ca. 4V, 50A) und einer Temperaturmeßeinrichtung befestigt. Das Aufnahmelager 3 ist über ein Scharnier 5 mit einem Gegenlager 6 verbunden. Beide Lager 3, 6 bestehen aus einem Isolierkörper, in dem eine langgestreckte Nut 7 ausgespart ist, die der Aufnahme eines Einmalartikels 8 dient, der von dem aufzuheizenden Fluid durchströmt wird.

Das Aufnahmelager 3 enthält ferner zwei voneinander beabstandete Stromzuführkonnektoren 9, die mit dem Elektroanschluß 4 in Verbindung stehen, wobei die Stromzuführkonnektoren 9 eine der Nut 7 ähnliche, jedoch querschnittlich größere Vertiefung aufweisen. Das klappbare Gegenlager 6 enthält an den den Stromzuführkonnektoren 9 entsprechenden Stellen Anpreßkontakte 10, deren im Querschnitt ebenfalls kreisbogenförmige Vertiefungen 11 in der Figur zu erkennen sind.

Vor dem in der Figur linken Stromzuführkonnektor 9 ist -an der in Fließrichtung stromaufwärtigen Seite- ein erster Thermistor 12 angeordnet, der die Temperatur der Flüssigkeit vor dem Einströmen in die Heizeinrichtung mißt. In Strömungsrichtung hinter dem anderen Stromzuführkonnektor 9 ist ein zweiter Thermistor 13 eingelassen, der die Temperatur der Flüssigkeit im Ausströmbereich der Heizeinrichtung mißt. Beide Thermistoren geben die von ihnen gemessenen Werte an eine nicht dargestellte Rechner- und Steuereinheit ab, die die Temperatur-Regelfunktion ausübt und die Durchflußmenge bzw. durchgeflossene Gesamtmenge errechnet.

Der in das Aufnahmelager 3 einzusetzende Einmalartikel 8 enthält an seinen beiden Enden Luer-Lock-Anschlüsse 14, 15, an die ein nicht dargestelltes Schlauchsystem anschließbar ist, ferner einen PVC-Schlauch 16, in den zwei Edelstahladapter 17 integriert sind, die mit dem in dem Einmalartikel 8 fließenden Fluid in Berührung stehen. Die PVC-Schlauchstücke 16a, 16b und 16c sowie die dazwischen angeordneten Edelstahladapter 17 werden so in die Nutabschnitte des Aufnahmelagers 3 eingesetzt, daß die Edelstahladapter 17 in Kontakt mit den Stromzuführkonnektoren 9 stehen. Wenn in diesem Zustand das Gegenlager 6 zugeklappt und mittels einer Verriegelung 18 mit dem Aufnahmelager 3 verriegelt wird, sind die beiden Edelstahladapter 17 fest gegen die Stromzuführkonnektoren 9 und die Anpreßkontakte 10 gepreßt, so daß ein einwandfreier elektrischer Kontakt gewährleistet ist.

Bei der Ausführung des erfindungsgemäßen Verfahrens wird das aufzuheizende Fluid durch den in der Figur linken Anschluß 14 in den PVC-Schlauch eingeführt, woraufhin die Eingangstemperatur von dem Thermistor 1 erfaßt wird. Mittels des über die Stromzuführkonnektoren 9 zugeführten Stromes wird das Fluid aufgeheizt, so daß es die vorgegebene, von dem Thermistor 13 an der Auslaufseite überwachte Temperatur erhält. Die hierzu aufgewendete Heizenergie wird exakt gemessen, wobei diese Meßwerte ebenfalls der Rechner- und Steuereinheit zugeführt werden.

Aus den die Temperatur und die aufgewendete Heizenergie betreffenden Meßwerten wird dann von der Rechnereinheit die Durchflußmenge des Fluids und die Gesamtdurchflußmenge errechnet.

**Patentansprüche**

1. Verfahren zum mengenmessen Aufheizen eines strömenden Fluids, insbesondere einer Flüssigkeit, die eine elektrische Heizeinrichtung durchströmt,
   **gekennzeichnet durch**
   folgende Schritte:
   Messen der Temperatur der Flüssigkeit vor dem Einströmen in die Heizeinrichtung und im Ausströmbereich der Heizeinrichtung,
   Bestimmen der Temperaturdifferenz,
   Messen der für den Heizenergieverbrauch der elektrischen Heizeinrichtung maßgeblichen Daten,
   Bestimmen des Heizenergieverbrauchs und
   Errechnen der Durchflußmenge aus der Temperaturdifferenz und dem Heizenergieverbrauch.

2. Verfahren nach Anspruch 1,
   dadurch gekennzeichnet, daß die nach einer vorbestimmten Zeitspanne durchgeflossene Gesamtdurchflußmenge m der Flüssigkeit und die Durchflußmenge pro Zeiteinheit aus folgenden Gleichungen errechnet werden:

4

$$(1) \qquad m = \frac{E}{c \times (T_0 - T_i)}$$

wobei

m = Gesamtdurchflußmenge
E = eingesetzte Heizenergie
c = fluidspezifische Konstante
$T_0$ = Temperatur am Ausgang der Heizeinrichtung
$T_i$ = Temperatur am Eingang der Heizeinrichtung

$$(2) \qquad m/t = \frac{P}{c \times (T_0 - T_i)}$$

wobei P = E/t ist.

3. Verfahren nach Anspruch 1 oder 2,
dadurch gekennzeichnet, daß die Heizeinrichtung von einem Computer in Abhängigkeit von der jeweils gemessenen Ausströmtemperatur der Flüssigkeit so geregelt wird, daß ein vorgegebener Temperatur-Sollwert eingehalten wird.

4. Verfahren nach einem der Ansprüche 1 bis 3,
dadurch gekennzeichnet, daß die Durchflußmenge/min und die gesamte Durchflußmenge mit vorgegebenen Grenzwerten verglichen werden und daß bei Überschreiten der Grenzwerte ein Alarmzeichen erzeugt wird.

5. Anordnung zum mengenmessenden Aufheizen eines strömenden Fluids, insbesondere einer Flüssigkeit, auf eine vorgegebene Temperatur, wobei die Flüssigkeit einen Kanal, insbesondere einen Schlauch, durchströmt, in den eine elektrische Heizeinrichtung integriert ist,
gekennzeichnet durch eine Einrichtung zur Erfassung der Temperaturerhöhung des Fluids,
eine Einrichtung zur Erfassung des Heizenergieverbrauchs der Heizeinrichtung und
eine Rechnereinheit zur rechnerischen Bestimmung der Durchflußmenge der Flüssigkeit aus den Daten der Temperaturerhöhung und des zugehörigen Heizenergieverbrauchs.

6. Anordnung nach Anspruch 5,
dadurch gekennzeichnet, daß ein Aufnahmelagerblock (13) ein Schlauchstück (8) aufnimmt und in Strömungsrichtung des aufzuheizenden Fluids einen ersten Thermistor (12) zur Erfassung der Eingangstemperatur des Fluids in dem Schlauch, zwei in Strömungsrichtung beabstandete, in einen Niedervolt-Regelstromkreis integrierte Stromzuführkonnektoren (9) zum Aufheizen des Fluids und einen zweiten Thermistor (13) zur Erfassung der Ausgangstemperatur des Fluids aufweist.

7. Anordnung nach Anspruch 5 oder 6,
dadurch gekennzeichnet, daß der Aufnahmelagerblock (3) mit einem Gegenlagerblock (6) scharnierartig verbunden ist und daß der Gegenlagerblock (6) an den Stromzuführkonnektoren (9) des Aufnahmelagerblocks (3) entsprechenden Stellen mit Anpreßkontakten (10) versehen ist.

8. Anordnung nach einem der Ansprüche 5 bis 7,
dadurch gekennzeichnet, daß das Schlauchstück (8) zwei mit dem strömenden Fluid in Kontakt stehende Edelstahladapter (17) aufweist, die in entsprechend geformte Mulden der Stromzuführkonnektoren (9) eingelegt werden und in der Schließstellung des Gegenlagerblocks (6) von dessen Anpreßkontakten (10) fest gegen die Stromzuführkonnektoren (9) gepreßt werden.

9. Anordnung nach einem der Ansprüche 5 bis 8,
dadurch gekennzeichnet, daß die Heizeinrichtung eine in den Schlauch eingefügte Heizkanüle ist, die über elektrische Kontaktklemmen in einen Niedervolt-Regelstromkreis integriert ist.

10. Anordnung nach einem der Ansprüche 5 bis 9,
ferner gekennzeichnet durch ein Anzeigegerät, das die Ausströmtemperatur der Flüssigkeit, die Durchflußmenge in g/min und die durchgeflossene Gesamtmenge in g anzeigt.

## EINSCHLÄGIGE DOKUMENTE

EP 91101866.1

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | US - A - 4 384 578 (H.E. WINKLER) * Gesamt * | 1,2 | A 61 M 5/44 A 61 M 1/36 G 01 F 1/68 |
| A | | 3,5,6, 7,8 | |
| | -- | | |
| X | DE - A1 - 3 112 144 (DEGUSSA AG) * Gesamt * | 1,2,5 | |
| A | | 3 | |
| | -- | | |
| X | DE - A1 - 3 006 584 (DEGUSSA AG) * Gesamt; insbesondere Fig. 3; Patentansprüche 1-4,6; Seite 3, Zeile 20 - Seite 4, Zeile 4 * | 1,2,5 | |
| A | | 3 | |
| | -- | | |
| X | JOURNAL OF PHYSICS E; Scientific instruments V., Band 21, Nr. 10, Oktober 1988 J.H. HUIJSING et al. "Thermal Mass-Flow Meter" Seiten 994-997 * Seite 994 * | 1,2 | |
| A | | 5 | |
| | -- | | |
| A | DE - A1 - 2 833 730 (D. ZEPPELIN) *. Fig. 3; Patentansprüche 1, 6,7,9,10; Seite 11, 1. u. 3. Absatz * | 1 | |
| | -- | | |
| A | DE - A1 - 2 802 993 (BAXTER LAB.) * Fig. 1-8; Seite 14, 5. Absatz - Seite 17, 1. Absatz; Seite 18, 2. Absatz - Seite 20, 3. Absatz * | 1 | |

RECHERCHIERTE
SACHGEBIETE (Int Cl⁵)

A 61 M
G 01 F

Der vorliegende Recherchenbericht wurde für alle Patentansprüche erstellt.

| Recherchenort WIEN | Abschlußdatum der Recherche 27-05-1991 | Prüfer LUDWIG |
|---|---|---|